# EUROPEAN PATENT APPLICATION

(11) **EP 4 775 972 A1**
(43) Date of publication of application: **15.07.2026**
(21) Application number: 26150052.4
(22) Date of filing: 02.01.2026
(51) Int. Cl.: G01N 27/414

(54) **RECEPTOR, SENSOR, SYNTHETIC SYSTEM, MEASUREMENT METHOD, AND RECEPTOR MANUFACTURING METHOD**

(30) Priority: 10.01.2025 JP 2025004262
(71) Applicant: Yokogawa Electric Corporation, Musashino-shi, Tokyo 180-8750 (JP); The University of Tokyo, Bunkyo-ku, Tokyo 113-8654 (JP)
(72) Inventor: OGAWA, Jun-Ichi, Musashino-shi, Tokyo, 180-8750 (JP); TORAI, Soichirou, Musashino-shi, Tokyo, 180-8750 (JP); TADENUMA, Takashi, Musashino-shi, Tokyo, 180-8750 (JP); MINAMI, Tsuyoshi, Bunkyo-ku, Tokyo, 113-8654 (JP); SASAKI, Yui, Bunkyo-ku, Tokyo, 113-8654 (JP); KAWASHIMA, Takayuki, Bunkyo-ku, Tokyo, 113-8654 (JP); OHSHIRO, Kohei, Bunkyo-ku, Tokyo, 113-8654 (JP)
(74) Representative: Hoffmann Eitle

(57) **Abstract**

Provided is a receptor comprising a base and a molecular imprinted polymeric film formed on the base and having a space that captures at least a part of a compound having an asymmetric carbon atom. The above-described receptor may comprise a metal layer formed between the base and the molecular imprinted polymeric film and having a nanostructure on a plane in contact with the molecular imprinted polymeric film. In any of the above-described receptor, the molecular imprinted polymeric film may have a noncovalently bonded functional group on a surface in which the space is formed.

## Description

### BACKGROUND

### 1. TECHNICAL FIELD

The present disclosure relates to a receptor, a sensor, a synthetic system, a measurement method, and a receptor manufacturing method.

### 2. RELATED ART

Patent document 1 describes that "a sensor which includes a molecularly imprinted polymer on a surface of the detection electrode can quantitatively detect a compound" (paragraph 0005). Patent document 2 describes that "chemically binding oxytocin antibody to a surface of a detection electrode via a linker and binding portion enables detection of oxytocin, which is the antigen" (paragraph 0005).

Non-Patent Document 1 describes that "The MIP films, which can specifically recognize and has an electrocatalytic effect on the oxidation of Trp and Tyr, together with the amplification function of an OECT, provide a highly sensitive and selective OECT biosensor" (Abstract).

### RELATED ART DOCUMENTS

### Patent Documents

Patent Document 1: Japanese Patent Application Publication No. 2023-61890
Patent Document 2: Japanese Patent Application Publication No. 2023-45665

### Non-Patent Documents

Non-Patent Document 1: Zhang, Lijun, et al., Chirality detection of amino acid enantiomers by organic electrochemical transistor., Biosensors and Bioelectronics 105 (2018): 121-128.
Non-Patent Document 2: Tsuyoshi Minami et al., Supramolecular Sensor for Cancer-Associated Nitrosamines, J. Am. Chem. Soc.134, 49 (2012): 20021-20024.

### SUMMARY

(1) In a first aspect of the present disclosure, provided is a receptor comprising a base and a molecular imprinted polymeric film formed on the base and having a space that captures at least a part of a compound having an asymmetric carbon atom.
(2) The above-described receptor in (1) may comprise a metal layer formed between the base and the molecular imprinted polymeric film and having a nanostructure on a plane in contact with the molecular imprinted polymeric film.
(3) In the above-described receptor in (1) or (2), the molecular imprinted polymeric film may have a noncovalently bonded functional group on a surface in which the space is formed. The noncovalently bonded functional group may be a hydrogen-bonded donor substituent.
(4) In the above-described receptor in (3), the molecular imprinted polymeric film may have three or more of the noncovalently bonded functional groups in one of the space.
(5) In any of the above-described receptors in (1) to (4), the molecular imprinted polymeric film may be composed of an aromatic monomer.
(6) In any of the above-described receptors (1) to (5), the chemical compound may include at least one of a heteroatom, an -OH group, or a -C=O group. The heteroatom may be at least one of an N atom, an O atom, an S atom, a P atom, a Cl atom, an I atom, or a Br atom.
(7) In any of the above-described receptors in (1) to (6), the chemical compound may have at least one of a primary amine group or a secondary amine group.
(8) In any of the above-described receptors in (1) to (7), the chemical compound may be at least one of histidine (His), a His derivative, a His analog, a side chain protector of His, or a peptide including His.
(9) In the above-described receptor in (5), the monomer may not include an alkyl group with of two or more carbon atoms as a substituent.
(10) The above-described receptor in (9), the monomer may include a hydrogen-bonded donor substituent as a substituent. The hydrogen-bonded donor substituent may be at least one of a hydroxyl group (-OH), an amino group (-NH₂, -NHR, -NR₂), an amide group (-CONH₂), a carboxy group (-COOH), a thiol group (-SH), an urea group (-NHCONH-), a guanidyl group (-C(=NH)-NH₂), a sulfonamide group (-SO₂NH₂), or an imino group (=NH).
(11) In the above-described receptor in (10), the monomer may be at least one of 1,2-diaminobenzene, 1,3-diaminobenzene, 1,4-diaminobenzene, 2-aminophenol, 1,3-dihydroxybenzene, or aniline.
(12) In a second aspect of the present disclosure, provided is a sensor comprising any of the above-described receptors in (1) to (11) and a field effect transistor having a gate connected to a metal in contact with the molecular imprinted polymeric film in the receptor.
(13) In a third aspect of the present disclosure, provided is a synthetic system comprising the above-described sensor in (12), a reactor, and a control unit which controls a reaction condition in the reactor based on a measurement result of the sensor.
(14) In a fourth aspect of the present disclosure, provided is a measurement method comprising bringing the receptor of the above-described sensor in (12) in contact with a sample including a compound, which is a detection target and performing measurement for a compound in the sample based on variation in current/voltage characteristic of the field effect transistor.
(15) The above-described measurement method in (14) may comprise performing quantitative measurement of the compound in the sample based on the variation in the current/voltage characteristic.
(16) In a fifth aspect of the present disclosure, provided is a method comprising performing measurement for the compound by using two or more of the above-described sensors in (12), the space of each of which captures different compounds and determining an optical purity of the compound based on a measurement result.
(17) In a sixth aspect of the present disclosure, provided is a receptor manufacturing method comprising mixing a compound including an asymmetric carbon atom with a monomer to prepare a monomer-containing liquid, applying the monomer-containing liquid on a base or immersing the base in the monomer-containing liquid, forming a polymer through polymerization of the monomer, and forming a molecular imprinted polymeric film by removing the compound.
(18) In the above-described receptor manufacturing method in (17), the polymerization may be electrolytic polymerization.
(19) In the above-described receptor manufacturing method in (17) or (18), the compound may be removed through electrochemical reaction.
(20) Any of the above-described receptor manufacturing methods in (17) to (19) may comprise forming a metal layer having a nanostructure on a surface of the base before the applying or the immersing.

Note that the summary clause of the invention does not necessarily describe all features of the present invention. In addition, the present invention may also be a sub-combination of the features described above.

### BRIEF DESCRIPTION OF THE DRAWINGS

Fig. 1 illustrates a cross-sectional view of a receptor 100 according to the present embodiment.
Fig. 2 illustrates a state where a metal film 125 is formed on a base 110 in a manufacturing method of the receptor 100 according to the present embodiment.
Fig. 3 illustrates a state where a nanostructure 130 is formed in the manufacturing method of the receptor 100 according to the present embodiment.
Fig. 4 illustrates a state where a polymer 400 is formed in the manufacturing method of the receptor 100 according to the present embodiment.
Fig. 5 illustrates a configuration of a sensor 500 according to the present embodiment, together with a sample 540.
Fig. 6 illustrates a configuration of a measurement apparatus 560 according to the present embodiment.
Fig. 7 illustrates a first example of an operational flow of the sensor 500 according to the present embodiment.
Fig. 8 illustrates a second example of the operational flow of the sensor 500 according to the present embodiment.
Fig. 9 illustrates a configuration of a sensor 900 according to a first modified example of the present embodiment, together with the sample 540.
Fig. 10 illustrates a configuration of a synthetic system 1000 according to a second modified example of the present embodiment.
Fig. 11 illustrates a configuration of a column 1100 according to a third modification example of the present embodiment.
Fig. 12 illustrates an example of a DPV measurement result using a receptor 100 in a present example.
Fig. 13 illustrates a comparative example of a DPV measurement result using a receptor 100 of a comparative example.
Fig. 14 illustrates an example of response specificity in the receptor 100 of the example.
Fig. 15 illustrates an example of a V_{g} - I_{d} curve in samples 540 with different optical purity.
Fig. 16 illustrates an example of a threshold voltage of the sample 540 with an optical purity of 80%ee or less.
Fig. 17 illustrates an example of a threshold voltage of the sample 540 with an optical purity of 80%ee or more.
Fig. 18 illustrates an example of a decision result of the optical purity using machine learning.
Fig. 19 illustrates an example of a computer 1200 in which a plurality of aspects of the present invention may be entirely or partially embodied.

### DESCRIPTION OF EXEMPLARY EMBODIMENTS

Hereinafter, the present invention will be described through embodiments of the invention, but the following embodiments do not limit the invention according to the claims. Also, not all of the combinations of features described in the embodiments are essential to the solving means of the invention.

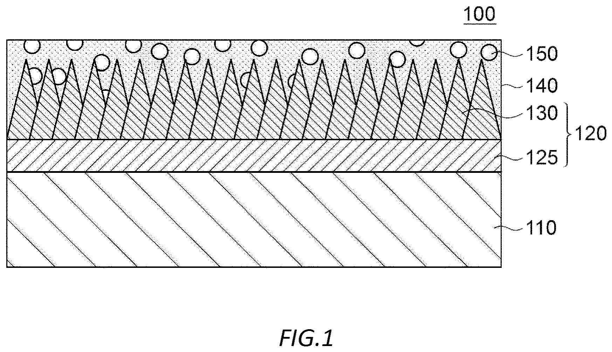

Fig. 1 illustrates a cross-sectional view of a receptor 100 according to the present embodiment. The receptor 100 is used as a detection electrode for detecting a compound having an asymmetric carbon atom. In the example of this figure, the receptor 100 includes a base 110, a metal layer 120, and a molecular imprinted polymeric film 140.

The base 110 has a face (a face on the upper side in this figure) in which each layer o ffthe receptor 100 is formed. In the present embodiment, the base 110 is plate-shaped. The base 110 may be formed with glass or resin. The base 110 may be formed with polyethylene naphthalate.

The molecular imprinted polymeric film 140 is formed on the base 110. Herein, being "on the base 110" means that the molecular imprinted polymeric film 140 is formed in a region on an upper side relative to the base 110, and means that the base 110 and the molecular imprinted polymeric film 140 may be in direct contact or the another layer may be included between the base 110 and the molecular imprinted polymeric film 140. The molecular imprinted polymeric film 140 has a space 150 which captures at least a part of a compound having an asymmetric carbon atom.

The molecular imprinted polymeric film 140 may have a noncovalently bonded functional group on a surface in which the space 150 is formed. The space 150 can thereby interact with and capture the at least a part of the compound. The noncovalently bonded functional group may be a hydrogen-bonded donor substituent. The hydrogen-bonded donor substituent may be at least one of a hydroxyl group (-OH), an amino group (-NH₂, -NHR, -NR₂), an amide group (-CONH₂), a carboxy group (-COOH), a thiol group (-SH), an urea group (-NHCONH-), a guanidyl group (-C(=NH)-NH₂), a sulfonamide group (-SO₂NH₂), or an imino group (=NH). For example, the hydrogen-bonded donor substituent may be a -OH group, a NH₂ group, or a -C≡N group. The molecular imprinted polymeric film 140 may have three or more noncovalently bonded functional groups in one space 150. The space 150 can thereby capture the compound more stably.

The molecular imprinted polymeric film 140 is formed by performing polymerization on one or more monomers. The molecular imprinted polymeric film 140 may be composed of an aromatic monomer. Therefore, the molecular imprinted polymeric film 140 may have repeating units including an aromatic moiety. By forming the molecular imprinted polymeric film from an aromatic monomer, rigidity of the space 150 is increased, which makes it easier for the space 150 to capture molecules more selectively. Through polymerization where the monomer is oxidized and a radical is generated, the molecular imprinted polymeric film 140 can be formed. The monomer may not include an alkyl group with of two or more carbon atoms as a substituent. By forming the molecular imprinted polymeric film 140 from such a monomer, the rigidity of the space 150 is increased, which makes it easier for the space 150 to capture the molecules more selectively. For example, the monomer may include the hydrogen-bonded donor substituent as a substituent. The hydrogen-bonded donor substituent may be at least one of a hydroxyl group (-OH), an amino group (-NH₂, -NHR, -NR₂), an amide group (-CONH₂), a carboxy group (-COOH), a thiol group (-SH), an urea group (-NHCONH-), a guanidyl group (-C(=NH)-NH₂), a sulfonamide group (-SO₂NH₂), or an imino group (=NH). The monomer may be at least one of 1,2-diaminobenzene, 1,3-diaminobenzene, 1,4-diaminobenzene, 2-aminophenol, 1,3-dihydroxybenzene, or aniline.

In the molecular imprinted polymeric film 140, for a first isomer and a second isomer having the same chemical formula, the percentage of capture amount of the first isomer to the total capture amount of the first isomer and the second isomer may be 60% or more. Alternatively, in the molecular imprinted polymeric film 140, the percentage of the capture amount of the first isomer to the total capture amount of the first isomer and the second isomer may be 70% or more, 80% or more, 90% or more, or 99% or more. By including such a molecular imprinted polymeric film 140, the receptor 100 can selectively capture the first isomer among a plurality of molecules having the same chemical formula.

The compound captured in the space 150 may have at least one of a heteroatom, a -OH group, or a -C=O group. Such a compound can easily be captured in the space 150. Herein, the heteroatom may be at least one of an N atom, an O atom, an S atom, a P atom, a Cl atom, an I atom, or a Br atom. The captured compound may have at least one of a primary amine group or a secondary amine group. Therefore, the N atom in the compound may be an N atom in the primary amine group or an N atom in the secondary amine group. The compound may be at least one of histidine (His), a His derivative, a His analog, a side chain protector of His, or a peptide including His.

The metal layer 120 is formed between the base 110 and the molecular imprinted polymeric film 140. The metal layer 120 may be formed of gold, aluminum, silver, copper, iron, titanium, or another metal material. Instead of the metal layer 120, a layer formed of indium tin oxide (ITO), poly(3,4-ethylenedioxythiophene), polystyrene sulfonate, a conductive carbon nanotube, graphene, or a conductive organic inorganic composite material may be formed between the base 110 and the molecular imprinted polymeric film 140. In the example of this figure, the metal layer 120 has a metal film 125 and a nanostructure 130. Alternatively, the metal layer 120 may not have the nanostructure 130.

The metal film 125 is formed on a face of the metal layer 120 that is in contact with the base 110. The metal film 125 is not particularly limited as long as it is in a film form.

The nanostructure 130 is formed in a face of the metal layer 120 that is in contact with the molecular imprinted polymeric film 140. The structure of the nanostructure 130 is not limited as long as the surface area of a face of the nanostructure 130 that is in contact with the molecular imprinted polymeric film 140 is greater than the surface area of the face where the metal film 125 is in contact with the base 110. The nanostructure 130 may be of a acicular structure which extends to the molecular imprinted polymeric film 140 side. The metal layer 120 having the nanostructure 130 allows the binding force between the metal layer 120 and the molecular imprinted polymeric film 140 to be increased compared to when the metal layer 120 does not have the nanostructure 130. The nanostructure 130 may be formed of a same material as the metal film 125, or may be formed of a different material from the metal film 125. Alternatively to the example of this figure, the base 110 and the metal film 125 may be formed of metal in a integrated manner.

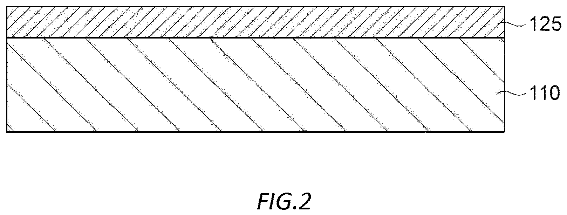

Figs. 2 to 4 illustrate examples of a manufacturing method of a receptor 100 according to the present embodiment. First, a base 110 is prepared, and a metal film 125 is formed on the base 110. Fig. 2 illustrates a state where a metal film 125 is formed on a base 110 in a manufacturing method of the receptor 100 according to the present embodiment. In this process, the metal film 125 may be formed on the base 110 through a sputtering method, a vacuum deposition method, or a plating method.

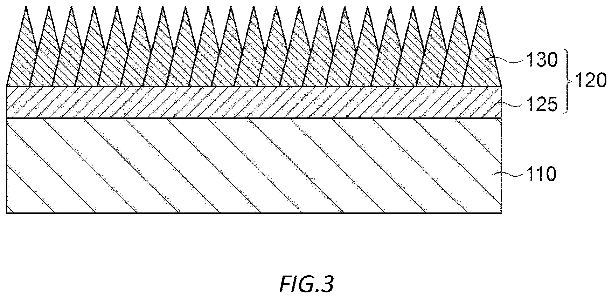

Then, a nanostructure 130 is formed on the metal film 125. Fig. 3 illustrates a state where a nanostructure 130 is formed in the manufacturing method of the receptor 100 according to the present embodiment. In this process, the nanostructure 130 may be formed on the metal film 125 by increasing or roughening the surface of the metal film 125. The nanostructure 130 may be formed on the metal film 125 through chronoamperometry using an aqueous solution of HAuCl₄. In this case, a concentration of the aqueous solution of HAuCl₄ may be approximately 100 mM. Through this process, the metal layer 120 having the nanostructure 130 on a surface of the base 110 is formed before application of or immersing in the monomer-containing liquid. By forming the metal layer 120 having the nanostructure 130 on the surface of the base 110, a binding force between the metal layer 120 and the polymer 400 or the molecular imprinted polymeric film 140 can be increased in a later process.

Then, the molecular imprinted polymeric film 140 is formed on the nanostructure 130. In this process, a compound 410 having an asymmetric carbon atom is mixed with a monomer to prepare a monomer-containing liquid. The molar ratio between the monomer and the compound 410 in the monomer-containing liquid may be 3:1 to 5:1. For example, the molar ratio between the monomer and the compound 410 in the monomer-containing liquid may be 4:1. The concentration of the monomer in the monomer-containing liquid may be 8 mM, and the concentration of the compound 410 may be 2 mM. Structural optimization calculation was performed on the mixture of the monomer and the compound 410 having different molar ratios by using density functional theory (DFT) calculation. The structural optimization calculation was performed with Gaussian 16 by using a basis set called B3LYP(D3BJ)/6-311G* and an IEFPCM model in an aqueous solution. Herein, "B3LYP" refers to a hybrid functional, and "D3BJ" refers to dispersion correction. "6-311G*" is a basis set, and "IEFPCM" refers to a continuum solvation model. Based on the molecular energy calculated by the structural optimization calculation, the degree of stabilization of the energy of the compound by the monomer was calculated. Specifically, the stabilization energy in the mixture of the compound and the composite was calculated by subtracting the energy of the compound alone and the energy of the monomer alone from the energy of a composite of the compound and the monomer and correcting the basis set superposition error (BSSE). The DFT calculation result of a case where the monomer is 1,2-diaminobenzene and the compound 410 is L-histidine is shown in Table 1.

**[Table 1]**

| MOLAR RATIO (1,2-DIAMINOBENZENE: L-HISTIDINE) | STABILIZATION ENERGY [kj/mol] |
|---|---|
| 3:1 | -128.9 |
| 4:1 | -155.6 |
| 5:1 | -166.2 |

According to Table 1, the mixture was better stabilized in a case where the molar ratio between the monomer and the compound 410 is 4:1, as compared to a case where the molar ratio is 3:1. According to Table 1, in a case where the molar ratio between the monomer and the compound 410 is 5:1, the mixture is better stabilized as compared to a case where the molar ratio is 4:1, but the percentage of variation in the stabilization degree relative to the increase in the number of monomers is smaller than a case where the molar ratio is 3:1. Therefore, in the case where the molar ratio between the monomer and the compound 410 is 4:1, the four monomers efficiently interact through hydrogen bonding with all the bonding portions of the template and the monomers and the compound 410 necessarily and sufficiently interact with each other. From the above, the molar ratio between the monomer and the compound 410 in the monomer-containing liquid is preferably approximately 4:1.

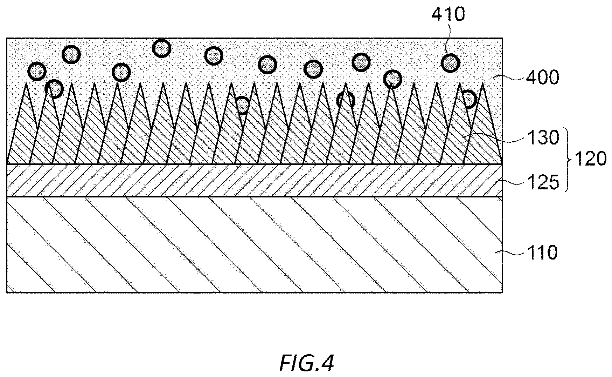

The monomer-containing liquid is applied on the base 110, or the base 110 is immersed in the monomer-containing liquid. The polymer 400 is formed by polymerizing the monomer. Fig. 4 illustrates a state where a polymer 400 is formed in the manufacturing method of the receptor 100 according to the present embodiment. The polymer 400 includes the compound 410. In this process, the polymerization may be electrolytic polymerization. The electrolytic polymerization may be performed by using cyclic Voltammetry. The electrolytic polymerization may be performed by repeating using the potential of a reference electrode as a reference to scan the potential applied between a working electrode and a counter electrode and allowing the potential to reach the potential at which chemical reaction is performed. The scanning of the potential may be performed repeatedly until the current no longer flows through the working electrode. The reference electrode may be an Ag/AgCl electrode. The working electrode may be the base 110 on which the metal layer 120 is formed. The counter electrode may be a platinum electrode. The potential applied between the working electrode and the counter electrode may be -0.5V to 1.5V, may be -0.5V to 1.0V, or may be 0.0V to 0.8V. The monomer is polymerized through the electrolytic polymerization, and thereby a uniform polymer 400 can be formed. Instead of the electrolytic polymerization, the monomer may be polymerized by adding a polymerization starter.

Then, the compound 410 is removed from the polymer 400 to form the molecular imprinted polymeric film 140. By removing the compound 410, a space 150 corresponding to the shape of the compound 410 is formed. In this process, the compound 410 may be removed through electrochemical reaction. The electrochemical reaction may be performed by using the cyclic Voltammetry in a basic solution. Removal of the compound 410 may be performed by repeating using the potential of the reference electrode as a reference to scan the potential applied between the working electrode and the counter electrode and allowing the potential to reach the potential at which the chemical bond is broken. The basic solution may be an aqueous solution of potassium hydroxide or sodium hydroxide. The basic solution may have a pH of 9 or more, 10 to 15, or 11 to 13. The reference electrode may be an Ag/AgCl electrode. The working electrode may be the base 110 on which the polymer 400 is formed. The counter electrode may be a platinum electrode. The potential applied between the working electrode and the counter electrode may be -1.0 V to 1.0 V. By removing the compound 410 through electrochemical reaction, the compound 410 can be sufficiently removed. Instead of the electrochemical reaction, the compound 410 may be removed by cleaning the polymer 400 or by immersing the polymer 400 in the basic solution.

According to the receptor 100 described above, the molecular imprinted polymeric film 140 having the space 150 that captures at least a part of the compound having an asymmetric carbon atom allows an enantiomer to be selectively captured.

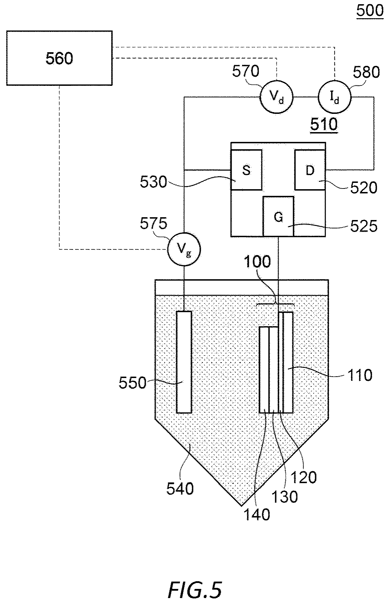

Fig. 5 illustrates a configuration of a sensor 500 according to the present embodiment, together with a sample 540. The sample 540 includes a compound that is the detection target. The sample 540 may further include a compound other than the detection target. The sample 540 may be a solution including the compound that is the detection target. For example, the sample 540 may be an aqueous solution including the compound that is the detection target.

The sensor 500 can detect the compound included in the sample 540. In the example of this figure, the sensor 500 includes a receptor 100, a field effect transistor 510, a reference electrode 550, a measurement apparatus 560, a first voltage source 570, a second voltage source 575, and a current measuring device 580.

The receptor 100 is brought into contact with the sample 540. The receptor 100 may be similar to the receptor 100 in Fig. 1.

The field effect transistor 510 may be a field effect transistor with a normal configuration, or may be an organic field effect transistor with an organic semiconductor. The field effect transistor 510 has a drain 520, a gate 525, and a source 530. The gate 525 is connected to metal in contact with the molecular imprinted polymeric film 140 in the receptor 100. The gate 525 may be connected to the metal layer 120.

The reference electrode 550 is connected to the source 530. The reference electrode 550 is brought into contact with the sample 540. The reference electrode 550 provides a stable potential, which enable an accurate measurement. The reference electrode 550 may be an Ag/AgCl electrode.

The measurement apparatus 560 is connected to the field effect transistor 510. The measurement apparatus 560 controls measurement by the sensor 500. The measurement apparatus 560 may be a computer such as a PC (personal computer), a tablet computer, a smartphone, a workstation, a server computer, or a general purpose computer, or may be a computer system in which a plurality of computers are connected. Such a computer system is also a computer in a broad sense. In addition, the measurement apparatus 560 may be implemented by one or more virtual computer environments that are executable in the computer. Alternatively, the measurement apparatus 560 may be a dedicated computer designed for the sensor 500, or may be a dedicated hardware achieved by the dedicated circuit.

The first voltage source 570 is connected to the measurement apparatus 560. The first voltage source 570 applies a voltage (V_{d}) between the source 530 and the drain 520 in the field effect transistor 510.

The second voltage source 575 is connected to the measurement apparatus 560. The second voltage source 575 applies a gate voltage (V_{g}) in the field effect transistor 510.

The current measuring device 580 is connected to the measurement apparatus 560. The current measuring device 580 detects a current (I_{d}) that flows through the source 530 and the drain 520 in the field effect transistor 510.

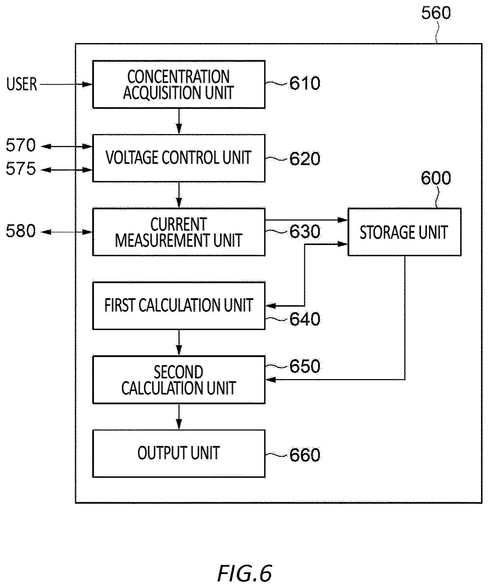

Fig. 6 illustrates a configuration of a measurement apparatus 560 according to the present embodiment. In the example of this figure, the measurement apparatus 560 includes a storage unit 600, a concentration acquisition unit 610, a voltage control unit 620, a current measurement unit 630, a first calculation unit 640, a second calculation unit 650, and an output unit 660.

The storage unit 600 stores measurement data of the sensor 500. The storage unit 600 may be an electronic storage medium, a magnetic storage medium, an optical storage medium, an electromagnetic storage medium, or a semiconductor storage medium. Although, in the example of this figure, the storage unit 600 is included in the measurement apparatus 560, it may be achieved by a storage region in at least a part of an external storage device such as a hard disk drive connected to the measurement apparatus 560, or may be achieved by an storage device external to the measurement apparatus 560, that is provided by a cloud sotrage service or the like, for example.

The concentration acquisition unit 610 acquires the concentration of the sample, when a sample with known concentration is measured. The concentration acquisition unit 610 may acquire the concentration of the sample based on an input by the user of the sensor 500. The concentration acquisition unit 610 may have an input/output circuit or a trasceiver circuit, and may perform data exchange with the user of the sensor 500 via an input/output apparatus (keyboard or the like and a display device or the like) used by the user of the sensor 500 or the like or a terminal apparatus used by the user of the sensor 500 or the like.

The voltage control unit 620 is connected to the concentration acquisition unit 610, the first voltage source 570, and the second voltage source 575. The voltage control unit 620 controls the V_{g} at the field effect transistor 510 via the first voltage source 570. The voltage control unit 620 controls the V_{d} at the field effect transistor 510 via the second voltage source 575. The voltage control unit 620 may cause the V_{g} to vary, while maintaining the V_{d} to be constant.

The current measurement unit 630 is connected to the storage unit 600, the voltage control unit 620, and the current measuring device 580. The current measurement unit 630 measures the I_{d} at the field effect transistor 510 via the current measuring device 580. The current measurement unit 630 stores the measurement data in the storage unit 600.

The first calculation unit 640 is connected to the storage unit 600. The first calculation unit 640 calculates a threshold voltage based on the measurement data stored in the storage unit 600. The first calculation unit 640 stores the calculated threshold voltage in the storage unit 600.

The second calculation unit 650 is connected to the storage unit 600 and the first calculation unit 640. The second calculation unit 650 calculates the concentration of the compound that is the detection target included in the sample, based on the threshold voltage stored in the storage unit 600 and the threshold voltage of the sample acquired from the first calculation unit 640.

The output unit 660 is connected to the second calculation unit 650. The output unit 660 performs a processing of displaying the concentration calculated by the second calculation unit 650 on a screen or the like. Herein, performing the processing of displaying on the screen is not limited to displaying on an actual screen on the display device, and includes generating display data to be displayed on a screen of a remote display device.

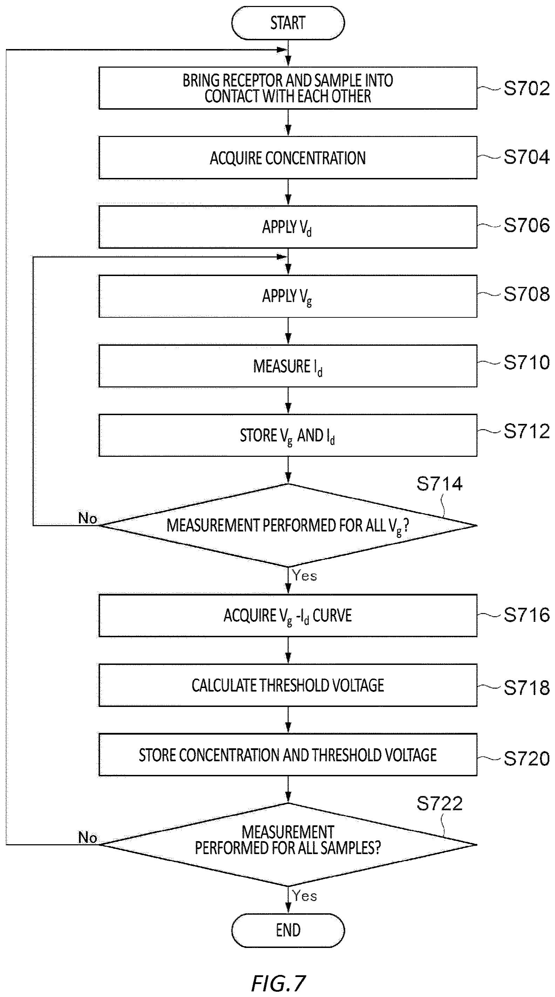

Fig. 7 illustrates a first example of an operational flow of the sensor 500 according to the present embodiment. This figure illustrates a flow of measuring, by the sensor 500, the sample with a known concentration of the compound that is the detection target. At step 702 (S702), the receptor 100 and the reference electrode 550 is brought into contact with the sample 540 including the compound that is the detection target. By separating the receptor 100 and the reference electrode 550 apart from each other and immersing them in the sample 540 in the form of a solution, the receptor 100 and the reference electrode 550 may be brought into contact with the sample 540. By bringing the receptor 100 into contact with the sample 540, the compound in the sample 540 is captured in the space 150 of the receptor 100.

At S704, the concentration acquisition unit 610 acquires the concentration of the compound included in the sample 540. T concentration acquisition unit 610 may acquire the concentration of the compound that is the detection target, based on an input by the user. Although S704 is performed between S702 and S706 in the example of this figure, it may be performed at any timing between S702 to S720.

At S706, the voltage control unit 620 applies V_{d} via the first voltage source 570. For example, V_{d} may be -1.0 V. At S708, the voltage control unit 620 applies V_{g} via the second voltage source 575. For example, V_{g} may be -0.5V to 3V.

At S710, the current measurement unit 630 measures the I_{d} via the current measuring device 580. At S712, the current measurement unit 630 acquires the concentration of the compound from the concentration acquisition unit 610, and acquires the V_{g} from the voltage control unit 620. The current measurement unit 630 stores the concentration of the compound, the V_{g}, and the I_{d} in the storage unit 600. The higher the concentration of the compound and the more compound is captured in the space 150, the lower the I_{d} becomes at the same V_{g}.

When the current measurement unit 630 does not measure the I_{d} for all the V_{g} (No at S714), the sensor 500 returns the processing to S708. At S708 after returning from S714, the voltage control unit 620 causes the V_{g} to vary. The voltage control unit 620 may increase or decrease the V_{g} at a constant interval (0.1 V or the like). At S710, the current measurement unit 630 measures the I_{d}. At S712, the current measurement unit 630 acquires the V_{g} after the variation from the voltage control unit 620, and stores the concentration of the compound, the V_{g}, and the I_{d} in the storage unit 600.

When the current measurement unit 630 measures the I_{d} for all the V_{g} (Yes at S714), the sensor 500 advances the processing to S716. At S716, the first calculation unit 640 acquires a V_{g} - I_{d} curve representing the relationship between the V_{g} and the I_{d} at said concentration. The first calculation unit 640 may acquire, from the storage unit 600, the concentration and a combination of the V_{g} and the I_{d} at said concentration, and generate the V_{g} - I_{d} curve at said concentration.

At S718, the first calculation unit 640 calculates the threshold voltage at said concentration. The higher the concentration of the compound becomes and the more compound is captured in the space 150, the threshold voltage moves to the negative direction. The first calculation unit 640 may calculate the threshold voltage by drawing an approximate straight line in a region (saturation region) where the relationship between a square root of I_{d} and the V_{g} becomes linear, and calculating a value of its X intercept. At S720, the first calculation unit 640 stores the concentration of the compound and the threshold voltage in the storage unit 600. The first calculation unit 640 may store the data in the same storage unit 600 as S712, or may store the data in a different storage unit 600 from S712.

When measurement for all the samples 540 with a known concentration of the compound are not performed (No at S722), the sensor 500 returns the processing to S702. At S702 after returning from S722, the receptor 100 and the reference electrode 550 are brought into contact with an unmeasured sample 540. When measurement for all the samples 540 with a known concentration of the compound are performed (Yes at S722), the sensor 500 ends the processing flow.

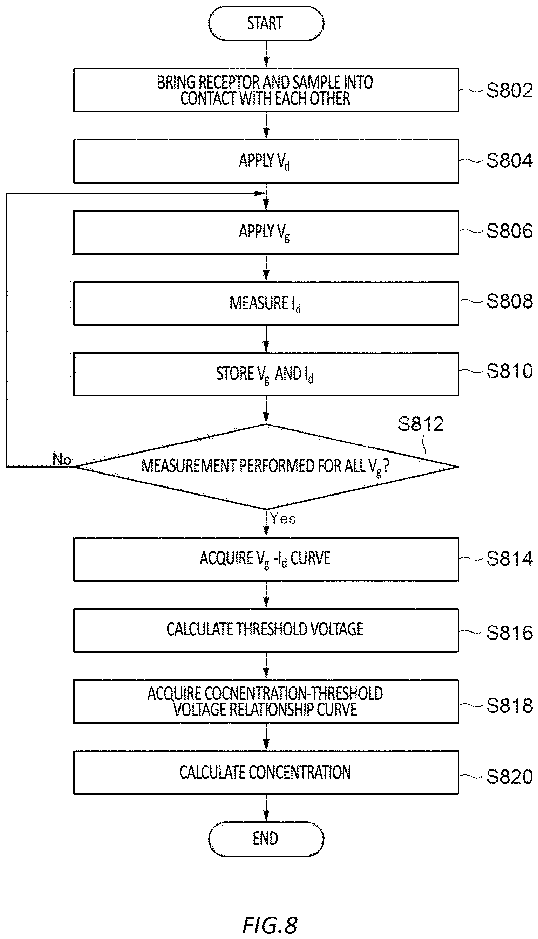

Fig. 8 illustrates a second example of the operational flow of the sensor 500 according to the present embodiment. Fig. 8 illustrates a flow of measuring the sample 540 with an unknown concentration of the compound by the sensor 500. At S802, the receptor 100 and the reference electrode 550 are brought into contact with the sample 540 including the compound that is the detection target. S802 may be similar to S702 in Fig. 7.

At S804, the voltage control unit 620 applies V_{d} via the first voltage source 570. For example, V_{d} may be -1.0 V. At S806, the voltage control unit 620 applies V_{g} via the second voltage source 575. For example, V_{g} may within -0.5V to 3V.

At S808, the current measurement unit 630 measures I_{d} via the current measuring device 580. At S810, the current measurement unit 630 acquires V_{g} from the voltage control unit 620. The current measurement unit 630 stores the V_{g} and the I_{d} in the storage unit 600.

When the current measurement unit 630 does not measure the I_{d} for all the V_{g} (No at S812), the sensor 500 returns the processing to S806. At S806 after returning from S812, the voltage control unit 620 causes the V_{g} to vary. The voltage control unit 620 may increase or decrease the V_{g} at a constant interval (0.1 V or the like). At S808, the current measurement unit 630 measures I_{d}. At S810, the current measurement unit 630 acquires the V_{g} after the variation from the voltage control unit 620, and stores the V_{g} and the I_{d} in the storage unit 600.

When the current measurement unit 630 measures the I_{d} for all the V_{g} (Yes at S812), the sensor 500 advances the processing to S814. At S814, the first calculation unit 640 acquires a V_{g} - I_{d} curve representing a relationship between the V_{g} and the I_{d} at said concentration. The first calculation unit 640 may acquire, from the storage unit 600, the concentration and a combination of the V_{g} and the I_{d} at said concentration, and generate the V_{g} - I_{d} curve at said concentration.

At S816, the first calculation unit 640 calculates a threshold voltage. The first calculation unit 640 may calculate the threshold voltage by drawing an approximate straight line in a region (saturation region) where the relationship between a square root of I_{d} and the V_{g} becomes linear, and calculating a value of its X intercept.

At S818, the second calculation unit 650 acquires a concentration-threshold voltage relationship curve. The second calculation unit 650 may acquire a combination of the concentration and a threshold voltage at said concentration from the storage unit 600 to generate the concentration-threshold voltage relationship curve. At S820, the second calculation unit 650 calculates the concentration of the compound included in the sample 540. The second calculation unit 650 may acquire, from the first calculation unit 640, the threshold voltage calculated at S816. The second calculation unit 650 may calculate the concentration of the compound included in the sample 540 from the threshold voltage calculated at S816 and the concentration-threshold voltage relationship curve. For example, the second calculation unit 650 may calculate the concentration in the concentration-threshold voltage relationship curve corresponding to a threshold voltage calculated at S816 as the concentration of the compound included in the sample 540. Therefore, the sensor 500 performs measurement for the compound in the sample 540 based on a variation in the current/voltage characteristic of the field effect transistor 510. The sensor 500 may perform quantitative measurement of the compound in the sample based on the variation in the current/voltage characteristic of the field effect transistor 510. The output unit 660 may output the concentration of the compound calculated by the second calculation unit 650.

According to the sensor 500 described above, a compound having an asymmetric carbon atom can be conveniently and rapidly detected. According to the sensor 500, the compound having an asymmetric carbon atom can be detected without any modification.

The measurement may be performed for the compound by using two or more sensors 500 with the space 150 capturing different compounds to determine an optical purity of the compound based on the measurement result. By using each of the two or more sensors 500 with the space 150 capturing different compounds, the concentration of each compound may be measured by the method described in Fig. 7 and Fig. 8. The optical purity of the compound may be determined based on the concentration of each compound calculated by each of the sensors 500. Each of the two or more sensors 500 may be different in the type of isomers that are captured, among a plurality of types of isomers with the same chemical formula. For example, a concentration of L-isomer in the sample 540 may be measured by using the sensor 500 including the space 150 that captures only the L-isomer, among the isomers, a concentration of D-isomer in the sample 540 may be measured by using the sensor 500 including the space 150 that captures only the D-isomer, among the isomers, and the optical purity of the compound may be determined based on the concentration of each of the L-isomer and the D-isomer.

Alternatively, measurement for the compound may be performed by using one sensor 500, to determine the optical purity of the compound based on the measurement result. The first calculation unit 640 of the measurement apparatus 560 may calculate the threshold voltage in each sample 540 by the method described in Fig. 7 and Fig. 8 by using a plurality of samples 540 for which the total amount of the compound that is the detection target and its isomers is constant and the optical purity of the compound that is the detection target is different. The second calculation unit 650 may acquire an optical purity-threshold voltage relationship curve by the method described for Fig. 8. The second calculation unit 650 may determine the optical purity of the sample 540 from the threshold voltage of the sample 540 having an unknown optical purity and a known total amount of the detection target and its isomers, based on the optical purity-threshold voltage relationship curve. T second calculation unit 650 may determine the optical purity from the optical purity-threshold voltage relationship curve by using support vector machine (SVM) regression. As an example, the second calculation unit 650 may perform SVM regression by using the method described in Non-Patent Document 2.

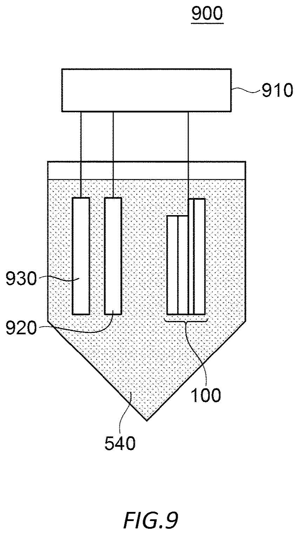

Fig. 9 illustrates a configuration of a sensor 900 according to a first modified example of the present embodiment, together with the sample 540. The sample 540 may be similar to that in Fig. 5. The sensor 900 can detect the compound included in the sample 540. In the example of this figure, the sensor 900 includes a receptor 100, a potentiostat 910, a counter electrode 920, and a reference electrode 930. The receptor 100 functions as the working electrode. The receptor 100 may be similar to that in Fig. 1.

The counter electrode 920 is brought into contact with the sample 540. The counter electrode 920 is a electrode in which a reaction that is equivalent to a redox reaction at the receptor 100, which is the working electrode, occurs. The counter electrode 920 may formed with a conductive material. The counter electrode 920 may be a metal electrode or a carbon electrode.

The reference electrode 930 is brought into contact with the sample 540. The reference electrode 930 provides a stable potential, which enables an accurate measurement. The reference electrode 930 may be an Ag/AgCl electrode.

The potentiostat 910 is connected to metal in contact with the molecular imprinted polymeric film 140 in the receptor 100, the counter electrode 920, and the reference electrode 930. The potentiostat 910 may be connected o the metal layer 120 in the receptor 100. The potentiostat 910 applies a voltage between the receptor 100 and the counter electrode 920, and controls the potential between the receptor 100 and the reference electrode 930. The potentiostat 910 may evaluate the current/voltage characteristic of the receptor 100 through differential pulse voltammetry (DPV). The sensor 900 may calculate the concentration of the compound included in the sample 540 based on the variation in the current value in the DPV.

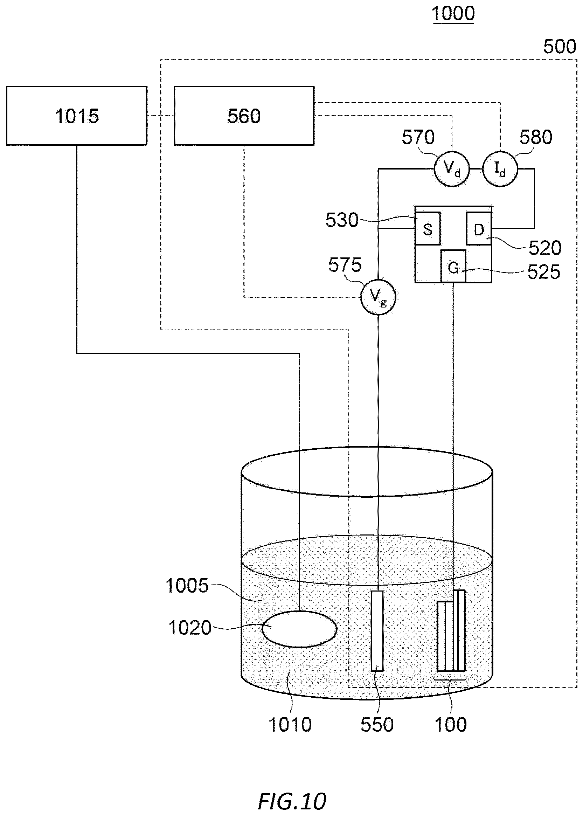

Fig. 10 illustrates a configuration of a synthetic system 1000 according to a second modified example of the present embodiment. The synthetic system 1000 is a system that performs a chemical reaction including the compound having an asymmetric carbon atom. In the example of this figure, the synthetic system 1000 includes one or more sensors 500, a reactor 1005, a control unit 1015, and a heater 1020.

The reactor 1005 is a container in which the chemical reaction including the compound having an asymmetric carbon atom is performed. In the example of this figure, the reactor 1005 is a vessel-type reactor. The reactor 1005 includes a solution 1010. The solution 1010 includes at least one of a reactant or a product. The solution 1010 includes a compound having an asymmetric carbon atom as either the reactant or the product. The solution 1010 may include the compound having the asymmetric carbon atom as the product.

The sensor 500 is brought into contact with the solution 1010. The sensor 500 performs a quantitative measurement of the compound having an isomer included in the solution 1010. The sensor 500 may be similar to that in Fig. 5. The sensor 500 may determine the optical purity of the compound included in the solution 1010. The synthetic system 1000 may include two or more sensors 500, and the optical purity of the compound included in the solution 1010 may be calculated based on the measurement result of the two or more sensors 500.

The control unit 1015 is connected to the measurement apparatus 560 of the sensor 500. The control unit 1015 acquires the measurement result of the sensor 500 from the measurement apparatus 560. The control unit 1015 controls the reaction condition at the reactor 1005 based on the measurement result of the sensor 500. The control unit 1015 may control the temperature of the reactor 1005 based on the optical purity of the compound included in the solution 1010 calculated from the measurement result of the one or more sensors 500. For example, the control unit 1015 may lower the temperature of the reactor 1005 when a measurement result that the optical purity of the compound included in the solution 1010 has fallen is acquired from the measurement apparatus 560. The control unit 1015 may control factors other than the temperature of the reactor 1005 (the volumeric flow rate, mixing ratio, agitation speed when the reactor 1005 includes an agitator, or the like).

The heater 1020 is connected to the control unit 1015. The control unit 1015 may control the temperature of the reactor 1005 via the heater 1020.

According to the synthetic system 1000 described above, the optical purity during the chemical reaction can be detected conveniently and rapidly, and the chemical reaction can be controlled based on the detected optical purity. Therefore, the synthetic system 1000 can control the optical purity of the product during the chemical reaction.

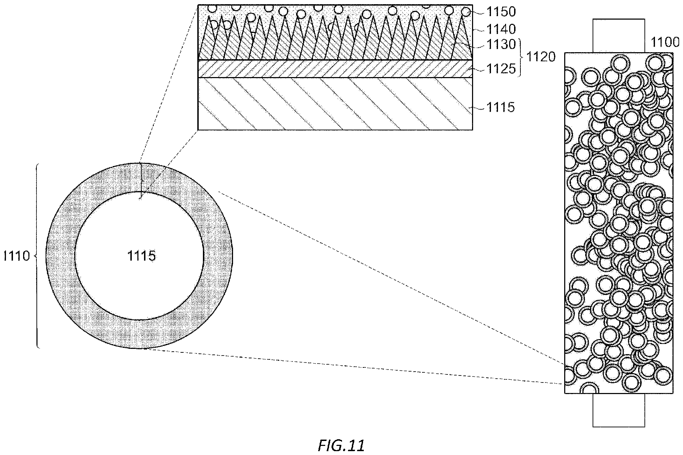

Fig. 11 illustrates a configuration of a column 1100 according to a third modification example of the present embodiment. The column 1100 dissociates the enantiomer by selectively capturing one type of enantiomer in the compound having an asymmetric carbon atom. In the example of this figure, the column 1100 has a cylindrical shape. The column 1100 includes a plurality of bulking agents 1110.

The bulking agent 1110 is filled in the column 1100. In the example of this figure, the bulking agent 1110 is spherical. Alternatively, the bulking agent 1110 may have a cylindrical, a cubic, a rectangular-cubic, or another solid geometry. The bulking agent 1110 include a base 1115, a metal layer 1120, and a molecular imprinted polymeric film 1140.

The base 1115 has an outer surface on which each layer of the bulking agents 1110 is formed. In the present embodiment, the base 1115 is spherical. Alternatively, the base 1115 may have a cylindrical, a cubic, a rectangular-cubic, or another solid geometry. The base 1115 may be formed of silica gel.

The molecular imprinted polymeric film 1140 is formed on the base 1115. The molecular imprinted polymeric film 1140 includes a space 1150 that captures at least a part of a compound having an asymmetric carbon atom. The molecular imprinted polymeric film 1140 may be similar to the molecular imprinted polymeric film 140 in Fig. 1, and the space 1150 may be similar to the space 150 in Fig. 1.

The metal layer 1120 is formed between the base 1115 and the molecular imprinted polymeric film 1140. The metal layer 1120 may be formed of a metal material similar to the metal layer 120 in Fig. 1. Instead of the metal layer 1120, a layer formed of indium tin oxide (ITO), poly(3,4-ethylenedioxythiophene), polystyrene sulfonate, a conductive carbon nanotube, graphene, or a conductive organic inorganic composite material may be formed between the base 1115 and the molecular imprinted polymeric film 1140. In the example of this figure, the metal layer 1120 includes a metal film 1125 and a nanostructure 1130. Alternatively, the metal layer 1120 may not include the nanostructure 1130. The metal film 1125 may be similar to the metal film 125 in Fig. 1, and the nanostructure 1130 may be similar to the nanostructure 130 in Fig. 1.

According to the column 1100 described above, since the space 1150 selectively captures the compound including the asymmetric carbon atom, the enantiomer can be dissociated by causing a fluid including the compound having the asymmetric carbon atom to flow therethrough.

Hereinafter, the present invention will be further described specifically based on examples, but the present invention is not limited to these examples.

### (Manufacture of the receptor 100)

### (Example)

The detection target is L-histidine, and the monomer is 1,2-diaminobenzene. Based on the method described in Fig. 2 to Fig. 4, the receptor 100 was manufactured by using a monomerenzen
-containing liquid with 1,2-diaminobenzene and L-histidine mixed at a molar ratio of:4:1.

### (Comparative Example)

The detection target is L-histidine, and the monomer is 1,2-diaminobenzene and dopamine. Based on the method described in Fig. 2 to Fig. 4, the receptor 100 was manufactured by using a monomer-containing liquid with 1,2-diaminobenzene, dopamine, and L-histidine mixed at a molar ratio of:1:4:1.

### (DPV measurement)

The sensor 900 in Fig. 9 was manufactured by using the receptor 100 of the Example or the Comparative Example. As the sample 540, a plurality of 100 mM of phosphate buffered saline (pH 6.0) including 5 mM of K₃Fe(CN)₆, 100 mM of KCl, and L-histidine was used. The measurement range was -0.05V to 0.8V (vs. Ag/AgCl).

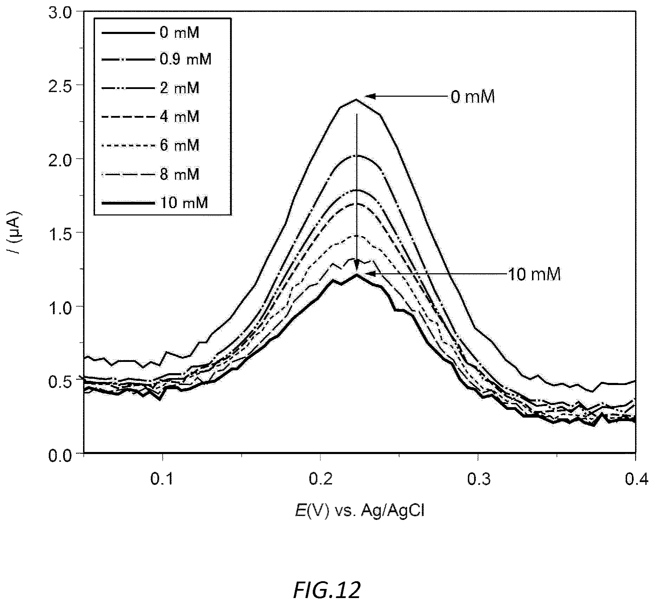

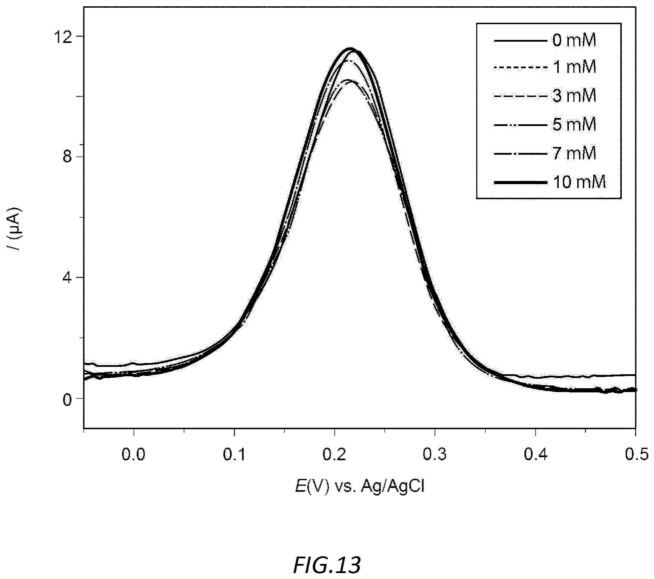

Fig. 12 and Fig. 13 illustrate the DPV measurement results. Fig. 12 illustrates an example of a DPV measurement result using a receptor 100 in the Example. The DPV measurement was performed by using the samples 540 with a L-histidine concentration of 0, 0.9, 2, 4, 6, 8, or 10 mM, respectively. In this figure, the peak near 0.22 V is caused by a redox reaction of K₃Fe(CN)₆ included in the sample 540. In this figure, the peak current value of the redox reaction of K₃Fe(CN)₆ was reduced as the concentration of L-histidine increased. Therefore, in the receptor 100 with 1,2-diaminobenzene as the monomer, a variation in the current/voltage characteristic according to the content of the L-histidine was observed.

Fig. 13 illustrates an example of a DPV measurement result using a receptor 100 of a comparative example. The DPV measurement was performed by using the samples 540 with a L-histidine concentration of 0, 1, 3, 5, 7, or 10 mM, respectively. Note that, the data obtained by using the sample 540 with a L-histidine concentration of 1 mM is overlapped with the data obtained by using the sample 540 with a L-histidine concentration of 3 mM. In this figure, the peak current value of the redox reaction of K₃Fe(CN)₆ was approximately constant even when the L-histidine concentration varied. In this manner, in the receptor 100 with 1,2-diaminobenzene and dopamine as monomers, any variation in the current/voltage characteristic according to the content of L-histidine was not observed. Therefore, in the present embodiment, by not including an alkyl group with two or more carbon atoms as a substituent in the monomer, the rigidity of the space 150 in the molecular imprinted polymeric film 140 was increased, making it easier for the space 150 to selectively capture the molecule.

### (Confirming response specificity)

The sensor 500 in Fig. 5 was manufactured by using the receptor 100 in the above-described example. A solution including 1 mM each of L-lysine (C₆H₁₄N₂O₂), L-tyrosine (C₉H₁₁NO₃), L-histidine (C₆H₉N₃O₂), D-histidine (C₆H₉N₃O₂), L-tryptophan (C₁₁H₁₂N₂O₂), or L-phenylalanine (C₉H₁₁NO₂) was used as the sample 540. Herein, L-histidine and D-histidine is in a relationship of isomers having the same chemical formula. For each of the samples 540, a threshold voltage (V_{TH}) was calculated by the method according to S702 to S718 in Fig. 7. By using, as the sample 540, a solution that does not include these compounds, the threshold voltage (V_{TH0}) of a reference was calculated with a similar approach.

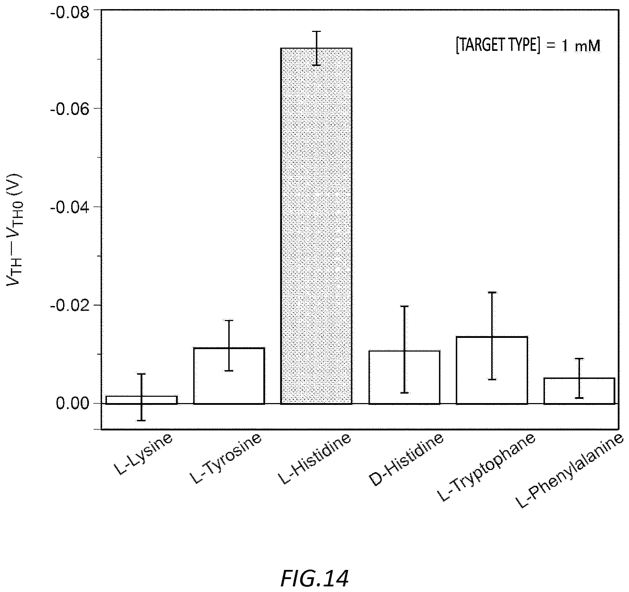

Fig. 14 illustrates an example of response specificity in the receptor 100 of the example. According to this figure, the difference between V_{TH} and V_{TH0} was about -.07 V in the measurement for the sample 540 including L-histidine, but the difference between V_{TH} and V_{TH0} was -0.015V or less in the measurement of the sample 540 including another compound. Therefore, in the receptor 100 of the Example, no variation in the current/voltage characteristic was observed for compounds and isomers with different chemical formula, and a variation in the current/voltage characteristic was observed only for L-histidine that is the detection target. From the above, the receptor 100 of the present invention was confirmed to specifically capture a particular molecule.

### (Calculation of optical purity)

The sensor 500 in Fig. 5 was manufactured by using the receptor 100 in the above-described example. As the sample 540, a plurality of 100 mM phosphate buffered saline (pH 6.0) including D-histidine and L-histidine was used. Herein, L-histidine and D-histidine is in a relationship of isomers having the same chemical formula. The total concentration of D-histidine and L-histidine in the sample 540 was 300µM, and the optical purity of histidine was -5.1%ee to 87.9%ee. For each of the samples 540, respective V_{g} - I_{d} curves were acquired by the method according to S702 to S716 in Fig. 7. A threshold voltage (V_{TH}) was calculated by the method according to S718 in Fig. 7. Herein, the measurement was performed with V_{d} of -2V and V_{g} in the range of -3V to 0.5V. By using the solution that does not include either D-histidine nor L-histidine as the sample 540, the threshold voltage (V_{TH0}) of the reference was calculated with a similar approach.

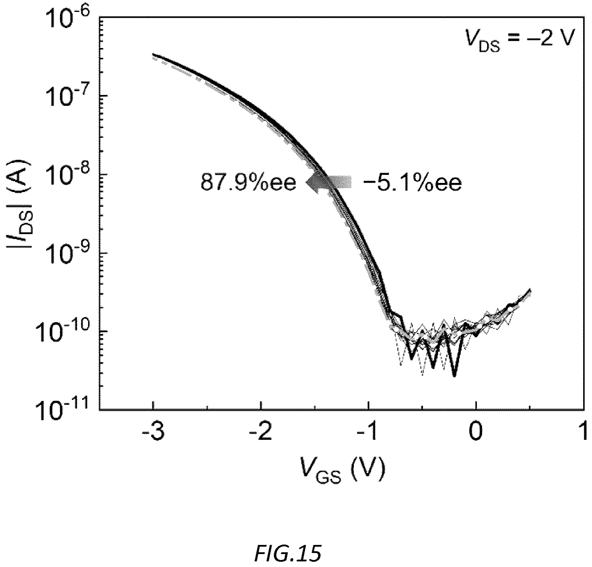

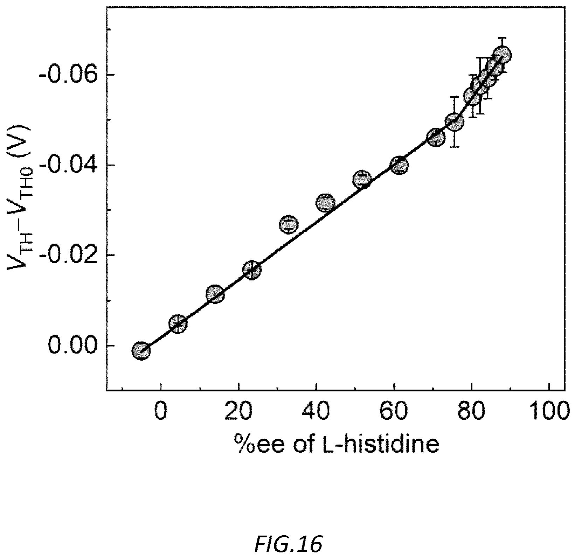

Fig. 15 to 18 illustrate measurement results of the sample 540 with different optical purity. Fig. 15 illustrates an example of a V_{g} - I_{d} curve in samples 540 with different optical purity. Fig. 16 illustrates an example of a threshold voltage of the sample 540 with an optical purity of 80%ee or less. In this figure, as the optical purity of L-histidine is increased, (V_{TH}-V_{TH0})/V_{TH} was increased linearly. Therefore, in the receptor 100, a variation in the current/voltage characteristic according to the optical purity of the compound that is the detection target, was observed.

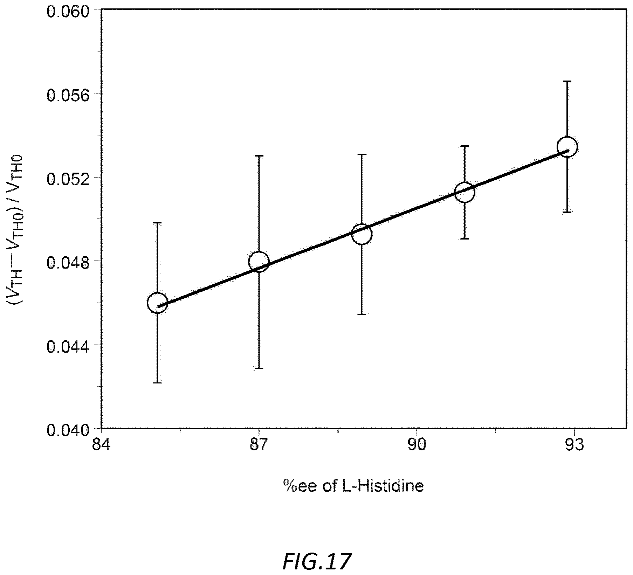

Fig. 17 illustrates an example of a threshold voltage of the sample 540 with an optical purity of 80%ee or more. Also in this figure, similarly to Fig. 16, as the optical purity of L-histidine is increased, (V_{TH}-V_{TH0})/V_{TH} was increased linearly. On the other hand, the gradient of (V_{TH}-V_{TH0})/V_{TH} in this figure was different from the gradient of (V_{TH}-V_{TH0})/V_{TH} in Fig. 16. Therefore, it was observed that the linear responsiveness of the threshold voltage to the optical purity is different depending on the region of the optical purity.

Based on the threshold voltage in each sample 540 of Fig. 16 and Fig. 17, the optical purity was determined by SVM regression. SVM regression was performed by using the method described in Non-Patent Document 2.

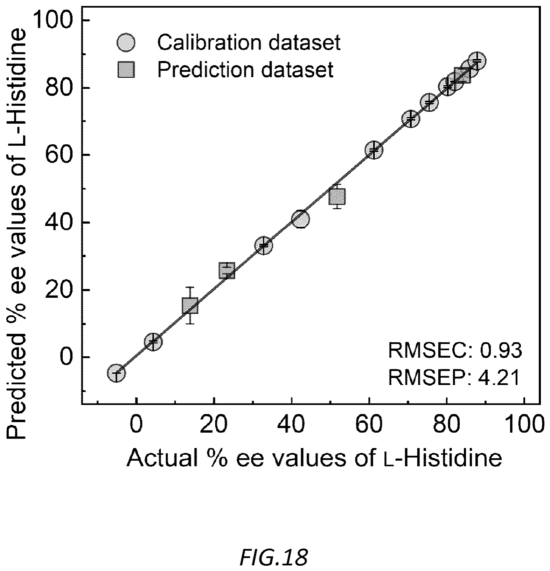

Fig. 18 illustrates an example of a decision result of the optical purity using machine learning. In this figure, a value of the optical purity calculated through SVM regression is indicated by the vertical axis, and an actual value of the optical purity is indicated by the horizontal axis. According to this figure, even though the linear responsiveness of the threshold voltage to the optical purity is different depending on the region of the optical purity, the optical purity can be accurately determined, for example, simultaneously at four points, by using SVM regression (in Fig. 18, the values of predicted ee% are 13.9 ee%, 23.4 ee%, 51.9 ee%, and 84.1 ee%). Therefore, as described in the present embodiment, by using a receptor 100 that targets only one optical isomer as the detection target, the sensor 500 can determine the optical purity of the detection target.

Various embodiments of the present invention may be described with reference to flowcharts and block diagrams, where blocks may represent (1) stages of processes in which operations are executed or (2) sections of apparatuses responsible for executing operations. Certain stages and sections may be implemented by a dedicated circuit, a programmable circuit supplied together with computer-readable instructions stored on computer-readable media, and/or processors supplied together with computer-readable instructions stored on computer-readable media. The dedicated circuit may include digital and/or analog hardware circuits, and may include integrated circuits (IC) and/or discrete circuits. The programmable circuit may include a reconfigurable hardware circuit including logical AND, logical OR, logical XOR, logical NAND, logical NOR, and other logical operations, a memory element or the like such as a flip-flop, a register, a field programmable gate array (FPGA) and a programmable logic array (PLA), or the like.

A computer-readable medium may include any tangible device that can store instructions to be executed by a suitable device, and as a result, the computer-readable medium having instructions stored thereon includes a product including instructions that can be executed in order to create means for executing operations specified in the flowcharts or block diagrams. Examples of the computer-readable medium may include an electronic storage medium, a magnetic storage medium, an optical storage medium, an electromagnetic storage medium, a semiconductor storage medium, and the like. More specific examples of the computer-readable medium may include a floppy (registered trademark) disk, a diskette, a hard disk, a random access memory, or RAM, a read-only memory, or ROM, an erasable programmable read-only memory, or EPROM or flash memory, an electrically erasable programmable read-only memory, or EEPROM, a static random access memory, or SRAM, a compact disc read-only memory, or CD-ROM, a digital versatile disk, or DVD, a Blu-ray (registered trademark) disk, a memory stick, an integrated circuit card, or the like.

The computer-readable instruction may include: an assembler instruction, an instruction-set-architecture (ISA) instruction; a machine instruction; a machine dependent instruction; a microcode; a firmware instruction; state-setting data; or either a source code or an object code described in any combination of one or more programming languages, including an object oriented programming language such as Smalltalk (registered trademark), JAVA (registered trademark), C++, or the like, and a conventional procedural programming language such as a "C" programming language or a similar programming language.

The computer-readable instruction may be provided for a processor or programmable circuit of a programmable data processing apparatus, such as a computer, locally or via a local area network (LAN), a wide area network (WAN) such as the Internet, or the like to execute the computer-readable instruction in order to create means for executing the operations specified in the flowcharts or block diagrams. Here, the computer may be a personal computer, or PC, a tablet computer, a smartphone, a workstation, a server computer, a general purpose computer, a special purpose computer, or the like, or may be a computer system to which a plurality of computers are connected. Such computer system to which the plurality of computers are connected is also referred to as a distributed computing system, and is a computer in a broad sense. In a distributed computing system, a plurality of computers collectively execute a program by each of the plurality of computers executing a portion of the program, and passing data during the execution of the program among the computers as needed.

Examples of the processor include a computer processor, a central processing unit (CPU), a processing unit, a microprocessor, a digital signal processor, a controller, a microcontroller, and the like. The computer may include one processor or a plurality of processors. In a multi-processor system including a plurality of processors, the plurality of processors collectively execute a program by each of the processors executing a portion of the program, and passing data during the execution of the program among the processors as needed. For example, in execution of multiple tasks, each of the plurality of processors may execute a portion of each task pieces by pieces by performing task-switching for each time slice. In this case, which portion of one program each processor is responsible for executing dynamically changes. Moreover, which portion of the program each of the plurality of processor is responsible for executing may be determined statically by multiprocessor-aware programming.

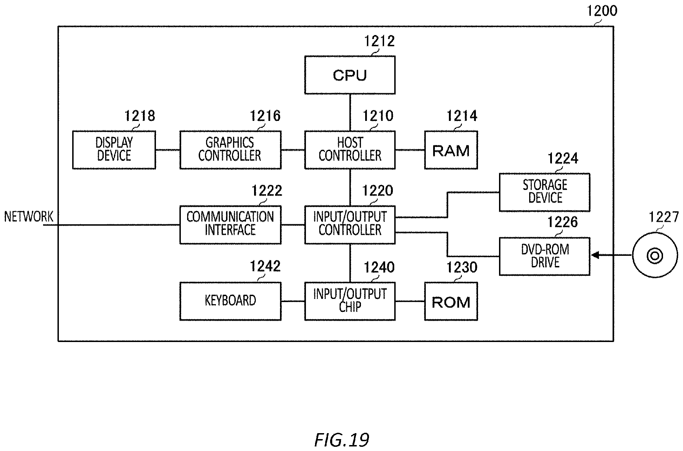

Fig. 19 shows an example of a computer 1200 in which a plurality of aspects of the present invention may be entirely or partially embodied. A program that is installed in the computer 1200 may cause the computer 1200 to function as operations associated with an apparatus according to the embodiment of the present invention or one or more sections in the apparatus, or may cause the computer 1200 to execute the operation or the one or more sections, and/or may cause the computer 1200 to execute processes according to the embodiment of the present invention or stages of the processes. Such a program may be executed by a CPU 1212 in order to cause the computer 1200 to execute particular operations associated with some or all of the blocks of flowcharts and block diagrams described herein.

The computer 1200 according to the present embodiment includes a CPU 1212, a RAM 1214, a graphics controller 1216, and a display device 1218, which are mutually connected by a host controller 1210. The computer 1200 also includes a communication interface 1222, a storage device 1224 such as a hard disk drive, input/output units such as a DVD-ROM drive 1226 and an IC card drive, which are connected to the host controller 1210 via an input/output controller 1220. The computer also includes legacy input/output units such as an ROM 1230 and a keyboard 1242, which are connected to the input/output controller 1220 via an input/output chip 1240.

The CPU 1212 operates according to programs stored in the ROM 1230 and the RAM 1214, thereby controlling each unit. The graphics controller 1216 acquires image data generated by the CPU 1212 on a frame buffer or the like provided in the RAM 1214 or in itself, and causes the image data to be displayed on a display device 1218.

The communication interface 1222 communicates with other electronic devices via a network. The storage device 1224 stores a program and data used by the CPU 1212 in the computer 1200. The DVD-ROM drive 1226 reads a program or data from a DVD-ROM 1227 and provides the program or data to the storage device 1224 via the RAM 1214. The IC card drive reads the programs and the data from the IC card, and/or writes the programs and the data to the IC card.

The ROM 1230 stores therein a boot program or the like that is executed by the computer 1200 at the time of activation, and/or a program which depends on the hardware of the computer 1200. The input/output chip 1240 may also connect various input/output units to the input/output controller 1220 via a parallel port, a serial port, a keyboard port, a mouse port, or the like.

Programs are provided by a computer-readable medium such as the DVD-ROM 1227 or the IC card. The programs are read from the computer-readable medium, are installed in the storage device 1224, the RAM 1214, or the ROM 1230, which are also an example of the computer-readable medium, and are executed by the CPU 1212. Information processing written in these programs is read by the computer 1200, and provides cooperation between the programs and the various types of hardware resources described above. An apparatus or method may be constructed by realizing the operation or processing of information according to the use of the computer 1200.

For example, when communication is executed between the computer 1200 and an external device, the CPU 1212 may execute a communication program loaded onto the RAM 1214 to instruct communication processing to the communication interface 1222, based on the processing described in the communication program. Under the control of the CPU 1212, the communication interface 1222 reads transmission data stored in a transmission buffer processing region provided in a recording medium such as the RAM 1214, the storage device 1224, the DVD-ROM 1227, or the IC card, transmits the read transmission data to the network, or writes reception data received from the network in a reception buffer processing region or the like provided on the recording medium.

In addition, the CPU 1212 may cause the RAM 1214 to read all or a necessary portion of a file or database stored in an external recording medium such as the storage device 1224, the DVD-ROM drive 1226, or the DVD-ROM 1227, the IC card, or the like, and may execute various types of processes on data on the RAM 1214. The CPU 1212 may then write back the processed data to the external recording medium.

Various types of information such as various types of programs, data, tables, and databases may be stored in a recording medium and subjected to information processing. The CPU 1212 may execute various types of processing on the data read from the RAM 1214, which includes various types of operations, information processing, conditional judging, conditional branch, unconditional branch, search/replace of information, or the like, as described throughout this disclosure and specified by an instruction sequence of programs, and writes the result back to the RAM 1214. **In** addition, the CPU 1212 may search for information in a file, a database, or the like in the recording medium. For example, when a plurality of entries, each having an attribute value of a first attribute associated with an attribute value of a second attribute, are stored in the recording medium, the CPU 1212 may search, from the plurality of entries, an entry with the attribute value of the first attribute specified that meets a condition, read the attribute value of the second attribute stored in said entry, and thereby acquiring the attribute value of the second attribute associated with the first attribute satisfying a predetermined condition.

The above-described program or software module may be stored in the computer-readable medium on the computer 1200 or near the computer 1200. In addition, a recording medium such as a hard disk or a RAM provided in a server system connected to a dedicated communication network or the Internet may be used as the computer-readable medium, thereby providing the program to the computer 1200 via the network.

While the present invention has been described above by way of the embodiments, the technical scope of the present invention is not limited to the scope described in the above-described embodiments. It is apparent to persons skilled in the art that various modifications or improvements can be made to the above-described embodiments. It is also apparent from description of the claims that the embodiments to which such modifications or improvements are made may be included in the technical scope of the present invention.

It should be noted that each process of the operations, procedures, steps, stages, and the like performed by the apparatus, system, program, and method shown in the claims, specification, or drawings can be executed in any order as long as the order is not indicated by "prior to", "before", or the like and as long as the output from a previous process is not used in a later process. Even if the operational flow is described using phrases such as "first" or "next" for the sake of convenience in the claims, specification, or drawings, it does not necessarily mean that the process must be performed in this order.

### EXPLANATION OF REFERENCES

100: receptor, 110: base, 120: metal layer, 125: metal film, 130: nanostructure, 140: molecular imprinted polymeric film, 150: space, 400: polymer, 410: compound, 500: sensor, 510: field effect transistor, 520: drain, 525: gate, 530: source, 540: sample, 550: reference electrode, 560: measurement apparatus, 570: first voltage source, 575: second voltage source, 580: current measuring device, 600: storage unit, 610: concentration acquisition unit, 620: voltage control unit, 630: current measurement unit, 640: first calculation unit, 650: second calculation unit, 660: output unit, 900: sensor, 910: potentiostat, 920: counter electrode, 930: reference electrode, 1000: synthetic system, 1005: reactor, 1010: solution, 1015: control unit, 1020: heater, 1100: column, 1110: bulking agent, 1115: base, 1120: metal layer, 1125: metal film, 1130: nanostructure, 1140: molecular imprinted polymeric film, 1150: space, 1200: computer, 1210: host controller, 1212: CPU, 1214: RAM, 1216: graphics controller, 1218: display device, 1220: input/output controller, 1222: communication interface, 1224: storage device, 1226: DVD-ROM drive, 1227: DVD-ROM, 1230: ROM, 1240: input/output chip, 1242: keyboard.

## Claims

1. A receptor comprising:
a base; and
a molecular imprinted polymeric film formed on the base and having a space that captures at least a part of a compound having an asymmetric carbon atom.

2. The receptor according to claim 1, comprising a metal layer formed between the base and the molecular imprinted polymeric film and having a nanostructure on a plane in contact with the molecular imprinted polymeric film.

3. The receptor according to claim 1 or 2, wherein the molecular imprinted polymeric film has a noncovalently bonded functional group on a surface in which the space is formed.

4. The receptor according to any one of claims 1 to 3, wherein the molecular imprinted polymeric film is composed of an aromatic monomer.

5. The receptor according to any one of claims 1 to 4, wherein the compound includes at least one of a heteroatom, an -OH group, or a -C=O group.

6. The receptor according to any one of claims 1 to 5, wherein the compound includes at least one of a primary amine group or a secondary amine group.

7. A sensor comprising:
the receptor according to any one of claims 1 to 6; and
a field effect transistor having a gate connected to a metal in contact with the molecular imprinted polymeric film in the receptor.

8. A synthetic system comprising:
the sensor according to claim 7;
a reactor; and
a control unit which controls a reaction condition in the reactor based on a measurement result of the sensor.

9. A measurement method comprising:
bringing the receptor of the sensor according to claim 7 in contact with a sample including a compound, which is a detection target; and
performing measurement for a compound in the sample based on variation in current/voltage characteristic of the field effect transistor.

10. The measurement method according to claim 9, comprising
performing quantitative measurement of the compound in the sample based on the variation in the current/voltage characteristic.

11. A method comprising:
performing measurement for the compound by using two or more sensors, each being identical to the sensor according to claim 7, the space of each of which captures different compounds; and
determining an optical purity of the compound based on a measurement result.

12. A receptor manufacturing method comprising:
mixing a compound including an asymmetric carbon atom with a monomer to prepare a monomer-containing liquid;
applying the monomer-containing liquid on a base or immersing the base in the monomer-containing liquid;
forming a polymer through polymerization of the monomer; and
forming a molecular imprinted polymeric film by removing the compound.

13. The receptor manufacturing method according to claim 12, wherein the polymerization is electrolytic polymerization.

14. The receptor manufacturing method according to claim 12 or 13, wherein the compound is removed through electrochemical reaction.

15. The receptor manufacturing method according to any one of claims 12 to 14, comprising forming a metal layer having a nanostructure on a surface of the base before the applying or the immersing.
